# EUROPEAN PATENT SPECIFICATION

(11) **EP 4 121 047 B1**
(45) Date of publication and mention of the grant of the patent: **30.10.2024**
(21) Application number: 21714661.2
(22) Date of filing: 18.03.2021
(51) Int. Cl.: A61K 31/4178, A61K 31/4418, A61K 45/06, A61P 9/10, A61P 11/00, A61P 13/12, A61P 19/02, A61P 37/00, C07D 409/10

(54) **IMIDAZOLYL THIOPHENE SULFONYL CARBAMATES FOR USE IN THE TREATMENT OF DISEASES ASSOCIATED WITH ANGIOTENSIN II**
IMIDAZOLYLTHIOPENSULFONYLCARBAMATE ZUR VERWENDUNG BEI DER BEHANDLUNG VON ERKRANKUNGEN IM ZUSAMMENHANG MIT ANGIOTENSIN II
CARBAMATES D'IMIDAZOLYLE THIOPHENE SULFONYLE DESTINÉS À ÊTRE UTILISÉS DANS LE TRAITEMENT DE MALADIES ASSOCIÉES À L'ANGIOTENSINE II

(30) Priority: 20.03.2020 GB 202004094
(43) Date of publication of application: 25.01.2023
(73) Proprietor: Vicore Pharma AB, 111 27 Stockholm (SE)
(72) Inventor: FEX, Tomas, SE 431 83 Mölndal (SE); OHLSSON, Bengt, SE 431 83 Mölndal (SE); RAUD, Johan, 111 27 Stockholm (SE)
(74) Representative: Potter Clarkson
(86) International application number: PCT/GB2021/050674
(87) International publication number: WO 2021/186180

(56) References cited:
- WO-A1-2016/139475
- WO-A1-2021/053344
- WO-A1-94/27597
- MATAVELLI LUIS C. ET AL: "AT2 Receptor Activities and Pathophysiological Implications", JOURNAL OF CARDIOVASCULAR PHARMACOLOGY, vol. 65, no. 3, 1 March 2016 (2016-03-01), US, pages 226 - 232, XP093102156, ISSN: 0160-2446, Retrieved from the Internet <URL:https://www.ncbi.nlm.nih.gov/pmc/articles/PMC4355033/pdf/nihms649987.pdf> DOI: 10.1097/FJC.0000000000000208
- YIQIAN WAN ET AL: "Design, Synthesis, and Biological Evaluation of the First Selective Nonpeptide AT 2 Receptor Agonist", JOURNAL OF MEDICINAL CHEMISTRY, vol. 47, no. 24, 1 November 2004 (2004-11-01), pages 5995 - 6008, XP055080127, ISSN: 0022-2623, DOI: 10.1021/jm049715t
- MAHALINGAM A K ET AL: "Selective angiotensin II AT"2 receptor agonists with reduced CYP 450 inhibition", BIOORGANIC & MEDICINAL CHEMISTRY, ELSEVIER, AMSTERDAM, NL, vol. 18, no. 12, 15 June 2010 (2010-06-15), pages 4570 - 4590, XP027072378, ISSN: 0968-0896, [retrieved on 20100409], DOI: 10.1016/J.BMC.2010.03.064

## Description

### Field of the Invention

This invention relates to the pharmaceutical use of certain compounds in medicine, as well as to novel pharmaceutically-active compounds, as defined in the claims. In particular, the invention relates to compounds for use in the treatment of a disease or condition in which activation of angiotensin II (Ang II) is desired or required, more particularly the treatment of a disease or condition in which activation of the Ang II type 2 (AT2) receptor is desired or required but in which inhibition of the cytochrome P450 enzymes (CYPs) is not desired, as defined in the claims.

In particular, it relates to the use of such compounds in the treatment of interstitial lung diseases.

### Background of the Invention

Renin, a protease, cleaves its only known substrate (angiotensinogen) to form angiotensin I (Ang I), which in turn serves as substrate to angiotensin converting enzyme (ACE) to form Ang II. The endogenous hormone Ang II is a linear octapeptide (Asp¹-Arg²-Val³-Tyr⁴-Ile⁵-His⁵-Pro⁷-Phe⁸), and is an active component of the renin angiotensin system (RAS). The angiotensin II type 1 (AT1) receptor is expressed in most organs, and is believed to be responsible for the majority of the pathological effects of Ang II.

Several studies in adult individuals appear to demonstrate that, in the modulation of the response following Ang II receptor stimulation, activation of the AT2 receptor has opposing effects to those mediated by the AT1. receptor. The AT2 receptor has also been shown to be involved in apoptosis and inhibition of cell proliferation (de Gasparo M et al., Pharmacol. Rev. (2000); 52, 415-472). More recently, AT2 receptor agonists have been shown to be of potential utility in the treatment and/or prophylaxis of disorders of the alimentary tract, such as dyspepsia and irritable bowel syndrome, as well as multiple organ failure (see international patent application WO 99/43339). The expected pharmacological effects of agonism of the AT2 receptor are described in general in de Gasparo M *et al., vide supra.*

The stimulating effects of Ang II on vascular tone, cell growth, inflammation and extracellular matrix synthesis are mainly coupled to the AT1 receptor in any organ, whereas the function of the AT2 receptor seems to be more prevalent in damaged tissue and exerts reparative properties and properties opposing the AT1 receptor. For example, the AT2 receptor has been shown to be of importance in relation to reduction of myocyte hypertrophy and fibrosis.

Interstitial lung diseases (ILDs) are a group of lung diseases that affect the interstitium, characterised by tissue around alveoli becoming scarred and/or thickened, and so inhibiting the respiratory process.

ILDs are distinct from obstructive airway diseases (e.g. chronic obstructive airway disease (COPD) and asthma), which are typically characterized by narrowing (obstruction) of bronchi and/or bronchioles. ILDs may be caused by injury to the lungs, which triggers an abnormal healing response but, in some cases, these diseases have no known cause. ILDs can be triggered by chemicals (silicosis, asbestosis, certain drugs), infection (e.g. pneumonia) or other diseases (e.g. rheumatoid arthritis, systemic sclerosis, myositis or systemic lupus erythematosus).

The most common ILDs are idiopathic pulmonary fibrosis (IPF) and sarcoidosis, both of which are characterised by chronic inflammation and reduced lung function.

Sarcoidosis is a disease of unknown cause that is characterised by collections of inflammatory cells that form lumps (granulomas), often beginning in the lungs (as well as the skin and/or lymph nodes, although any organ can be affected). When sarcoidosis affects the lungs, symptoms include coughing, wheezing, shortness of breath, and/or chest pain.

Treatments for sarcoidosis are patient-specific. In most cases, symptomatic treatment with non-steroidal anti-inflammatory drugs (NSAIDs) is possible, but for those presenting lung symptoms, glucocorticoids (e.g. prednisone or prednisolone), anti metabolites and/or monoclonal anti-tumor necrosis factor antibodies are often employed.

IPF is a lung-disease of unknown cause that affects about 5 million people globally. It has no curative treatment options except, in rare cases, lung transplantation, resulting in a chronic, irreversible, progressive deterioration in lung function and, in most cases, leading to death within 2-5 years (median survival 2.5 to 3.5 years). While the overall prognosis is poor in IPF, it is difficult to predict the rate of progression in individual patients. Risk factors for IPF include age, male gender, genetic predisposition and history of cigarette smoking. The annual incidence is between 5-16 per 100,000 individuals, with a prevalence of 13-20 cases per 100,000 people, increasing dramatically with age (King Jr TE et al., Lancet (2011); 378, 1949-1961; Noble PW et al., J. Clin. Invest. (2012); 122, 2756-2762). IPF is limited to the lungs and is recalcitrant to therapies that target the immune system which distinguishes it from pulmonary fibrosis associated with systemic diseases.

Patients with IPF usually seek medical assistance due to chronic and progressive exertional dyspnea and cough. Imaging of the lung classically reveals traction bronchiectasis, thickened interlobar septae and subpleural honeycombing. When all three manifestations are present and there is no evidence of a systemic connective tissue disease or environmental exposure, a diagnosis of IPF is very likely. A definite diagnosis is usually made by lung biopsy and requires a multidisciplinary team of expertise including pulmonologists, radiologists and pathologists experienced in interstitial lung diseases.

IPF demonstrates different phenotypes with different prognosis, defined as mild, moderate and severe. Mild cases follow a stable or slow progressive path with patients sometimes taking several years to seek medical advice. Accelerated IPF has a much more rapid progression with shortened survival, affecting a sub-group of patients, usually male cigarette smokers. Acute exacerbations of IPF are defined as a rapid worsening of the disease, and patients in this sub-population have very poor outcomes with a high mortality rate in the short run. The cause of IPF is unknown but it appears to be a disorder likely arising from an interplay of environmental and genetic factors resulting in fibroblast driven unrelenting tissue remodeling rather than normal repair; a pathogenesis primarily driven by fibrosis rather than inflammation. A growing body of evidence suggests that the disease is initiated through alveolar epithelial cell microinjuries and apoptosis, activating neighboring epithelial cells and attracting stem or progenitor cells that produce the factors responsible for the expansion of the fibroblast and myofibroblast populations in a tumor like way. The fibroblastic foci secrete exaggerated amounts of extracellular matrix that destroys the lung parenchyma and ultimately leads to loss of lung function.

The mean annual rate of decline in lung function (vital capacity) is within a range of 0.13-0.21 litres. Symptoms precede diagnosis by 1-2 years and radiographic signs may precede symptoms (Ley B et al., Am. J. Respir. Crit. Care Med. (2011); 183,431-440).

Numerous treatment approaches have been tested in pre-clinical models and clinical trials such as anti-inflammatory, immune-modulatory, cytotoxic, general anti-fibrotic, anti-oxidant, anti-coagulant, anti-chemokine, anti-angiogenic drugs as well as RAS-blockers, endothelin antagonists, and sildenafil, all of which have basically been shown to provide limited or no benefits (Rafii R et al., J. Thorac. Dis. (2013); 5, 48-73).

Current treatment of IPF includes oxygen supplementation. Medications that are used include pirfenidone or nintedanib, but only with limited success in slowing the progression of the disease. Further, both of these drugs commonly cause (predominantly gastrointestinal) side-effects.

There are drawbacks associated with all of the aforementioned ILD (and IPF) drug treatments and there is a real clinical need for safer and/or more effective treatments.

To restore the alveolar epithelium is very desirable as a therapeutic effect in IPF, and therefore stem cell therapy has also been tested. Some preclinical studies have shown promise in the use of pluripotent stem cells that can differentiate into lung epithelial and endothelial cells, thereby repairing lung injury and fibrosis.

Currently, a lung transplant is the only intervention that substantially improves survival in IPF patients. However, complications such as infections and transplant rejection are not uncommon.

The development of new treatment strategies for IPF is therefore important. Thus, the fundamental challenge for the future is to develop appropriate therapeutic approaches that will reverse or stop the progression of the disease.

US patent application US 2004/0167176 describes the preparation of tricyclic heterocycles useful as Ang II receptor agonists.

Selective AT2 receptor agonists with reduced CYP 450 inhibition are described in Mahalingam et al., Bioorg. Med. Chem. (2010); 18, 4570-4590.

Transesterification methods for synthesis of AT2 receptor ligands with improved stability in human liver microsomes are described in Wannberg et al., Bioorg. Med. Chem. Lett. (2018); 28, 519-522.

In particular, international patent application WO 2002/096883 describes the preparation of imidazolyl, triazolyl, and tetrazolyl thiophene sulfonamides and derivatives as AT2 receptor agonists. Of the compounds described in that document (as Example 1) is the compound C21 (*N*-butyloxycarbonyl-3-(4-imidazol-1-ylmethylphenyl)-5-isobutylthiophene-2-sulfon-amide). C21 was selected for clinical development from a group of about 20 related analogues as a selective AT2 receptor agonist (see in particular Wan et al, J. Med. Chem. (2004) 47, 5995-6008). It is now in clinical development for treatment of AT2 receptor related disorders, including IPF (see, for example, international patent application WO 2016/139475).

C21 has also been indicated to be of potential use in the treatment of *inter alia,* stroke, spinal cord injury, sickle cell disease, muscular dystrophy, cancer treatment-related cardiotoxicity, peripheral neuropathy and systemic sclerosis (see, for example, international patent applications WO 2004/046141, WO 2016/092329, WO 2016/107879, WO 2016/139475, WO 2017/221012, WO 2019/008393, and US patent application US 2012/035232).

It has been found during development that C21 has the disadvantage that it is both a potent inhibitor of several Cytochrome P450 enzymes (CYPs), especially CYP 2C9 and CYP 3A4, potentially affecting the metabolism of other drugs, and also rapidly hydrolysed to an inactive sulfonamide metabolite.

It is thus a fundamental challenge to develop potent and selective AT2 agonists that are stable metabolically and/or exhibit less inhibition of CYP enzymes. We have found, surprisingly, that certain chemically-modified compounds as defined hereinafter are not only selective AT2 receptor agonists but are also more potent, have a significantly improved stability to metabolic hydrolysis and/or exhibit less inhibition of CYP enzymes, compared to C21.

### Description of the Invention

The references to methods of treatment in this description are to be interpreted as references to the compounds, pharmaceutical compositions and medicaments of the present invention for use in a method for treatment of the human (or animal) body by therapy.

We have now surprisingly found that certain AT2 receptor agonists are useful in those conditions in which activation of the AT2 receptor is desired or required but in which inhibition of CYPs is not desired, such as interstitial lung diseases.

Unless indicated otherwise, all technical and scientific terms used herein have the same meaning as commonly understood by one of ordinary skill in the art to which this invention pertains.

In a first aspect of the invention, there is provided a compound of formula I, 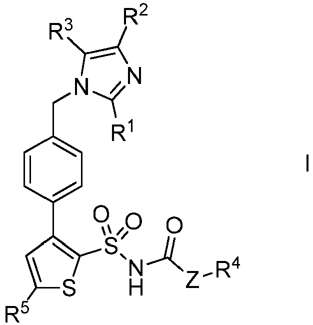 wherein:
Z represents -O- or a direct bond;
R¹ represents C₁₋₃ alkyl, optionally substituted by one or more halogen atoms;
R² and R³ each independently represent H or C₁₋₃ alkyl, optionally substituted by one or more halogen atoms;
R⁴ represents C₁₋₆ alkyl or C₁₋₆ alkoxy, each of which is optionally substituted with one or more halogen atoms, or
R⁴ represents aryl, C₁₋₆ alkylaryl, heteroaryl or C₁₋₆ alkylheteroaryl, each of which is optionally substituted by one or more substituents selected from halogen, -CF₃, -OCF₃, C₁₋₆ alkyl, and C₁₋₆ alkoxy;
R⁵ represents C₁₋₆ alkyl, C₁₋₆ alkoxy or C₁₋₆ alkoxy-C₁₋₆ alkyl, each of which is optionally substituted by one or more halogen atoms,
or a pharmaceutically-acceptable salt thereof, for use in the treatment of a disease or condition in which activation of AT2 receptors is desired or required but in which inhibition of one or more CYP enzymes is not desired, wherein said disease or condition is selected from the group consisting of an interstitial lung disease, an autoimmune disease, a chronic kidney disease, pulmonary hypertension, an obstructive airway disease, a viral respiratory tract infection and pneumonia as a consequence thereof and infarction.

By a 'disease or condition in which activation of AT2 receptors is desired or required but in which inhibition of CYPs is not desired', we include diseases or conditions that are known to be treatable by activation of AT2 receptors, such as those mentioned hereinafter, but wherein existing treatments of such conditions may comprise administration of other therapeutic agents that are metabolized by CYPs. Such diseases or conditions may thus include conditions in which inhibition of at least one CYP enzyme is not required, advantageous and/or desirable, or in which such inhibition is or would be detrimental to the patient.

The aformentioned compounds (including pharmaceutically acceptable salts) that are disclosed herein for use in the treatment of a disease or condition in which activation of AT2 receptors is desired or required but in which inhibition of CYPs is not desired, wherein said disease or condition is selected from the group consisting of an interstitial lung disease, an autoimmune disease, a chronic kidney disease, pulmonary hypertension, an obstructive airway disease, a viral respiratory tract infection and pneumonia as a consequence thereof and infarction, are referred to together hereinafter as the 'compounds of the invention'.

There is further provided (not part of the claimed invention) the use of a compound of formula I, or a pharmaceutically acceptable salt thereof, in the manufacture of a medicament for use in the treatment of a disease or condition in which activation of AT2 receptors is desired or required but in which inhibition of CYPs is not desired, wherein said disease or condition is selected from the group consisting of an interstitial lung disease, an autoimmune disease, a chronic kidney disease, pulmonary hypertension, an obstructive airway disease, a viral respiratory tract infection and pneumonia as a consequence thereof and infarction.

There is further provided a method of treating a disease or condition in which activation of AT2 receptors is desired or required but in which inhibition of CYPs is not desired, wherein said disease or condition is selected from the group consisting of an interstitial lung disease, an autoimmune disease, a chronic kidney disease, pulmonary hypertension, an obstructive airway disease, a viral respiratory tract infection and pneumonia as a consequence thereof and infarction, comprising administering to a patient in need of a therapeutically effective amount of a compound of formula I, or a pharmaceutically acceptable salt thereof.

For the avoidance of doubt, the skilled person will understand that references herein to compounds of particular aspects of the invention (such as any aspect of the invention referring to compounds of formula I as defined hereinbefore) will include references to all embodiments and particular features thereof, which embodiments and particular features may be taken in combination to form further embodiments and features of the invention.

Unless indicated otherwise, all technical and scientific terms used herein will have their common meaning as understood by one of ordinary skill in the art to which this invention pertains.

As described herein, the compounds of the invention are useful because they possess pharmacological activity, and/or are metabolised in the body following oral or parenteral administration to form compounds that possess pharmacological activity. In particular, compounds of the invention are agonists of AT2 receptors. Compounds of the invention are thus expected to be useful in those conditions in which an increase in the activity of AT2 receptors is desired or required.

More particularly, compounds of the invention are agonists of the AT2 receptor, and, especially, are selective (vs. the AT1 receptor) agonists of that sub-receptor, for example as may be demonstrated in the tests described below.

AT2 receptor agonists include those that fully, and those that partially, activate the AT2 receptor. Compounds of the invention may thus bind selectively to the AT2 receptor, and exhibit agonist activity at the AT2 receptor. By compounds that 'bind selectively" to the AT2 receptor, we include that the affinity ratio for the relevant compound (AT2:AT1) at a given concentration is at least 50:1, such as at least 100:1, preferably at least 1000: 1.

The compounds of the invention are further expected to be useful in those conditions where AT2 receptors are expressed and their stimulation is desired or required.

In this respect, compounds of the invention are indicated in the treatment of conditions characterised by vasoconstriction, fibrosis, increased cell growth and/or differentiation, increased cardiac contractility, increased cardiovascular hypertrophy, and/or increased fluid and electrolyte retention, as well as skin disorders and musculoskeletal disorders.

Particular diseases or condition in which activation of AT2 receptors is desired or required but in which inhibition of CYPs is not desired are interstitial lung diseases (e.g. pulmonary fibrosis, IPF, systemic sclerosis and sarcoidosis), autoimmune diseases (e.g. rheumatoid arthritis, systemic lupus erythematosus, multiple sclerosis, psoriasis and inflammatory bowel disease), chronic kidney diseases (e.g. diabetic nephropathy), pulmonary hypertension, pulmonary arterial hypertension and/or infarction (e.g. myocardial infarction and stroke). Thus, compounds of the invention are particularly useful in the treatment of interstitial lung diseases, such as IPF; autoimmune diseases, such as rheumatoid arthritis; chronic kidney diseases, such as diabetic nephropathy; pulmonary hypertension, including pulmonary arterial hypertension; and/or infarction, such as myocardial infarction.

Compounds of the invention are particularly indicated in the treatment and/or prevention of ILDs, such as sarcoidosis or fibrosis, more specifically pulmonary fibrosis and particularly IPF, as well as conditions that may trigger ILDs, such as systemic sclerosis, rheumatoid arthritis, myositis or systemic lupus erythematosus, or are otherwise associated with ILDs, such as pulmonary hypertension and/or pulmonary arterial hypertension.

Compounds of the invention are particularly useful in the treatment of pulmonary fibrosis, in particular IPF.

There is further provided a method of treatment of pulmonary fibrosis, and in particular IPF, which method comprises administration of a therapeutically effective amount of a compound of the invention to a person suffering from such a condition.

In the treatment of pulmonary fibrosis, including IPF, compounds of the invention may have an anti-fibrotic effect, with reduction of fibrosis and prevention of further deposition of extracellular matrix. Compounds of the invention may reduce lung scarring/wound healing and also have an anti-apoptotic effect, thereby preventing apoptosis of alveolar endothelial cells, being an initiating factor for the development of pulmonary fibrosis. Compounds of the invention may also have an anti-proliferative effect, thus reducing the cancer-like proliferation of fibroblasts and myofibroblasts in pulmonary fibrosis. Compounds of the invention may also improve vascular remodelling in pulmonary fibrosis, thereby reducing secondary pulmonary hypertension. Finally, compounds of the invention may demonstrate anti-inflammatory, anti-growth factor (e.g. transforming growth factor beta) and/or anti-cytokine effects.

In addition, compounds of the invention may also be useful in the treatment or prevention of any fibrotic condition of one or more internal organs characterised by the excessive accumulation of fibrous connective tissue, and/or in the treatment or prevention of fibrogenesis and the morbidity and mortality that may be associated therewith. Such fibrosis may be associated with an acute inflammatory condition, such as acute respiratory distress syndrome (ARDS), severe acute respiratory syndrome (SARS), and multiple-organ inflammation, injury and/or failure, which may be caused by internal or external trauma (e.g. injury), or by an infection.

Such conditions may thus result from sepsis or septic shock caused by a viral, bacterial or fungal infection (e.g. viral respiratory tract infection). Furthermore, acute lung injury, ARDS and, particularly, SARS may be caused by viruses, such as coronaviruses, include the novel SARS coronavirus 2 (SARS-CoV-2), which may result in internal tissue damage, dysfunction of relevant internal (e.g. mucosal) tissues, such as the respiratory epithelium, and so result in virally-induced pneumonia, impaired lung function, respiratory dysfunction, distress and/or failure. Such tissue damage may also give rise to severe fibrosis. Such tissue damage may also give rise to severe fibrosis. For example, the SARS disease caused by the novel coronavirus SARS-CoV-2 (coronavirus disease 2019 or COVID-19) is known in many cases to result in fibrosis.

There is further provided a method of treatment of a disease or condition in which activation of AT2 receptors is desired or required but in which inhibition of CYPs is not desired (such as pulmonary fibrosis, in particular IPF), wherein said disease or condition is selected from the group consisting of an interstitial lung disease, an autoimmune disease, a chronic kidney disease, pulmonary hypertension, an obstructive airway disease, a viral respiratory tract infection and pneumonia as a consequence thereof and infarction, which method comprises administration of a therapeutically effective amount of a compound of the invention to a person suffering from the relevant condition.

The compounds of the invention are indicated both in the therapeutic, palliative, and/or diagnostic treatment, as well as the prophylactic treatment (by which we include preventing and/or abrogating deterioration and/or worsening of a condition) of any of the above conditions.

Compounds of the invention will normally be administered orally, intravenously, subcutaneously, buccally, rectally, dermally, nasally, tracheally, bronchially, by any other parenteral route, or *via* inhalation or pulmonary route, or any combination thereof, in a pharmaceutically acceptable dosage form, in solution, in suspension, in emulsion, including nanosuspensions, or in liposome formulation. Additional methods of administration include, but are not limited to, intraarterial, intramuscular, intraperitoneal, intraportal, intradermal, epidural, intrathecal administration, or any combination thereof.

In some embodiments, the compounds of the invention may be administered alone (e.g. separately), and/or sequentially, and/or in parallel at the same time (e.g. concurrently), using different administrative routes, but are preferably administered by way of known pharmaceutical formulations, including tablets, capsules or elixirs for oral administration, suppositories for rectal administration, sterile solutions, suspensions or emulsions for parenteral or intramuscular administration, or *via* inhalation, and the like. Administration through inhalation is preferably done by using a nebulizer, thus delivering the compound of the invention to the small lung tissue including the alveoli and bronchioles, preferably without causing irritation or cough in the treated subject.

Preferably, administration of a therapeutically effective amount of a compound of the invention is performed by a combination of administrative routes, either separately (e.g. about 2 or more hours apart from one another), sequentially (e.g. within about 2 hours of one another), or in parallel at the same time (e.g. concurrently), including *via* inhalation and orally, achieving an effective dosage.

There is further provided a method of treating a disease or condition in which activation of AT2 receptors is desired or required (and such diseases or conditions in which inhibition of CYPs is not desired), wherein said disease or condition is selected from the group consisting of an interstitial lung disease, an autoimmune disease, a chronic kidney disease, pulmonary hypertension, an obstructive airway disease, a viral respiratory tract infection and pneumonia as a consequence thereof and infarction, including pulmonary fibrosis, and in particular IPF, which method comprises administering a therapeutically effective amount of a compound of the invention through a combination of administrative routes, either separately, sequentially, or in parallel at the same time, preferably *via* inhalation and orally, in order to achieve effective amount or dosage, to a patient in need of such a therapy.

Such combinations of administrative routes, preferably *via* inhalation and orally, may be presented as separate formulations of the compound of invention that are optimized for each administrative route.

Such formulations may be prepared in accordance with standard and/or accepted pharmaceutical practice.

Depending upon the patient to be treated and the route of administration, the compounds of the invention may be administered at varying doses. Although doses will vary from patient to patient, suitable daily doses are in the range of about 0.1 to about 1000 mg (e.g. 0.1, 0.5, 1, 2, 5, 10, 15, 20, 25, 30, 35, 40, 45, 50, 75, 100, 150, 200, 250, 300, 350, 400, 450, 500, 550, 600, 650, 700, 750, 800, 850, 900, 950, 1000 mg, and the like, or any range or value therein) per patient, administered in single or multiple doses. More preferred daily doses are in the range of about 0.1 to about 250 mg (e.g., 0.2, 0.3, 0.4, 0.5, 1, 1.5, 2, 2.5, 3, 3.5, 4. 4.5, 5, 5.5, 6, 6.5, 7, 7.5, 8, 8.5, 9, 9.5, 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250 mg, and the like, or any range or value therein) per patient. A particular preferred daily dose is in the range of from about 0.3 to about 100 mg per patient.

Individual doses of compounds of the invention may be in the range of about 0.1 to about 100 mg (e.g. 0.3, 0.5, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100 mg, and the like, or any range or values therein).

In any event, the physician, or the skilled person, will be able to determine the actual dosage, which will be most suitable for an individual patient, which is likely to vary with the condition that is to be treated, as well as the age, weight, sex and response of the particular patient to be treated. The above-mentioned dosages are exemplary of the average case; there can, of course, be individual instances where higher or lower dosage ranges are merited, and such are within the scope of this invention.

The benefits of using the compounds of the invention, preferably *via* a combination of administrative routes, separately, and/or sequentially, and/or in parallel at the same time is to produce a tailored treatment for the patient in need of the therapy, with the possibility of preventing and/or reducing side effects, and also tune the correct dosage levels of a therapeutically effective amount of a compound of the invention.

Subjects suitable to be treated with formulations of the present invention include, but are not limited to, mammalian subjects, in particular human subjects.

Compounds of the invention find particular utility when combined with other therapeutic agents in combination therapy to treat the various conditions, including those mentioned hereinbefore. Because compounds of the invention exhibit minimal CYP enzyme inhibition, such combinations are particularly advantageous when the other therapeutic agents that are employed for use in the relevant condition are themselves metabolized by CYP enzymes.

Thus, when the condition to be treated is an interstitial lung disease, such as IPF, systemic sclerosis or fibrotic diseases that are known in the art, compounds of the invention are preferably administered in combination with a Galectin-3 inhibitor, a lysophosphatidic acid receptor 1 (LPA1) antagonist, an autotaxin (ATX) inhibitor, a recombinant human pentraxin-2 protein or established therapies for such treatment, including but not limited to pirfenidone and/or nintedanib. Preferably, the combination of compound of the invention is with pirfenidone, or a pharmaceutically-acceptable salt thereof, which compound is known to be metabolized by CYP enzymes, such as CYP1A.

Further, when the condition to be treated is a chronic kidney related disease, compounds of the invention are preferably administered in combination with one or more other drugs that are also used in such treatments, such as irbesartan and/or torsemide, which compounds are known to be metabolized by CYP enzymes, such as CYP2C9.

When the condition to be treated is pulmonary hypertension, compounds of the invention are preferably administered in combination with one or more other drugs that are also used in such treatment, such as selexipag and/or sildenafil, which compounds are known to be metabolized by CYP enzymes, such as CYP3A4.

When the condition to be treated or prevented is myocardial infarction and/or a stroke-related disease, compounds of the invention are preferably administered in combination with one or more other drugs that are also used in such treatment, such as propranolol, warfarin, clopidogrel, atorvastatin, cilostazol, lidocaine and/or simvastatin, or a pharmaceutically-acceptable salt thereof, which compounds are known to be metabolized by CYP enzymes, such as CYP1A, CY2CP and/or CYP3A4.

When the condition to be treated is an autoimmune disease, such as rheumatoid arthritis, multiple sclerosis or psoriasis, compounds of the invention are preferably administered in combination with one or more other drugs that are also used in such treatment, including but not limited to non-steroidal anti-inflammatory drugs (NSAIDs), such as naproxen, celecoxib, meloxicam or an analogue thereof (e.g. piroxicam) orindomethacin; or a drug such as tizanidine, cyclophosphamide, cyclosporine, deflazacort and/or hydrocortisone, riluzole, or a pharmaceutically-acceptable salt thereof, which compounds are known to be metabolized by CYP enzymes, such as CYP1A, CYP2CP, CYP2C19 and/or CYP3A4.

Thus, compounds of the invention are particularly useful in the treatment of a disease or condition in which activation of the AT2 receptor is desired or required but in which inhibition of CYPs is not desired and so may be administered to treat diseases, including those mentioned hereinbefore, in which in combination with one or more of the other therapeutic agents mentioned hereinbefore, which are metabolized through a CYP enzyme pathway, is or may be useful, including pirfenidone, naproxen, propranolol, riluzole, tizanidine, warfarin, celecoxib, clopidogrel, irbesartan, meloxicam, piroxicam, torsemide, cyclophosphamide, indomethacin, atorvastatin, cilostazol, cyclosporine, deflazacort, hydrocortisone, lidocaine, selexipag, sildenafil and/or simvastatin. Most preferably, the compounds of the invention are administered in combination with pirfenidone to treat an interstitial lung disease, such as IPF.

Therapeutic agents that may be used in conjunction with compounds of the invention include variously-applied standard treatments for viral infections, including antibody therapies (e.g. LY-CoV555/LY-CoV016 (bamlanivimab and etesevimab), LY-CoV555 (bamlanivimab, Eli Lilly), REGN-COV2 (casirivimab and imdevimab), REGN3048-3051, TZLS-501, SNG001 (Synairgen), eculizumab (Soliris; Alexion Pharmaceuticals), ravulizumab (Ultomiris; Alexion Pharmaceuticals), lenzilumab, leronlimab, tocilizumab (Actemra; Roche), sarilumab (Kevzara; Regeneron Pharma), and Octagam (Octapharma)), including antiviral medicines (e.g. oseltamivir, remdesivir, favilavir, molnupiravir, simeprevir, daclatasvir, sofosbuvir, ribavirin, umifenovir, lopinavir, ritonavir, lopinavir/ritonavir (Kaletra; AbbVie Deutschland GmbH Co. KG), teicoplanin, baricitinib (Olumiant; Eli Lilly), ruxolitinib (Jakavi; Novartis), tofacitinib (Xeljanz; Pfizer), the TMPRSS2 inhibitor camostat, or camostat mesylate, Actemra (Roche), AT-100 (rhSP-D), MK-7110 (CD24Fc; Merck)), OYA1 (OyaGen9), BPI-002 (BeyondSpring), NP-120 (Ifenprodil; Algernon Pharmaceuticals), and Galidesivir (Biocryst Pharma), antiinflammatory agents (e.g. NSAIDs, such as ibuprofen, ketorolac, naproxen, and the like), chloroquine, hydroxychloroquine, interferons (e.g. interferon beta (interferon beta-1a), tocilizumab (Actemra), lenalidomide, pomalidomide and thalidomide), analgesics (e.g. paracetamol or opioids), antitussive agents (e.g. dextromethorphan), vaccinations (e.g. INO-4800 by Inovio Pharmaceuticals and Beijing Advaccine Biotechnology, if available), COVID-19 convalescent plasma (CCP) and/or passive antibody therapy with antibodies from blood of people who have recovered from infection with SARS-CoV or SARS-CoV-2.

Further therapeutic agents that may be mentioned include anti-fibrotics (e.g. nintedanib and, particularly, pirfenidone), vitamins (e.g. vitamin B, C and D) and mucolytics such as acetylcysteine and ambroxol.

Other therapeutic agents that may be used in conjunction with compounds of the invention or pharmaceutically acceptable salts thereof in accordance with the invention include corticosteroids. Corticosteroids include both naturally-occurring corticosteroids and synthetic corticosteroids.

Naturally-occurring corticosteroids that may be mentioned include cortisol (hydrocortisone), aldosterone, corticosterone, cortisone, pregnenolone, progesterone, as well as naturally-occurring precursors and intermediates in corticosteroid biosynthesis, and other derivatives of naturally-occurring corticosteroids, such as 11-deoxycortisol, 21-deoxycortisol, 11-dehydi-ocorticosterone, 11-deoxycorticosterone, 18-hydroxy-11-deoxycorticosterone, 18-hydroxycorticosterone, 21-deoxycortisone, 11β-hydroxypregnenolone, 11β,17α,21-trihydroxypregnenolone, 17α,21-dihydroxypregnenolone, 17α-hydroxypregnenolone, 21-hydroxypregnenolone, 11-ketoprogesterone, 11β-hydroxyprogesterone, 17a-hydroxyprogesterone and 18-hydroxyprogesterone.

Synthetic corticosteroids that may be mentioned include those of the hydrocortisone-type (Group A), such as cortisone acetate, hydrocortisone aceponate, hydrocortisone acetate, hydrocortisone buteprate, hydrocortisone butyrate, hydrocortisone valerate, tixocortol and tixocortol pivalate, prednisolone, methylprednisolone, prednisone, chloroprednisone, cloprednol, difluprednate, fludrocortisone, fluocinolone, fluperolone, fluprednisolone, loteprednol, prednicarbate and triamcinolone; acetonides and related substances (Group B), such as amcinonide, budesonide, desonide, fluocinolone cetonide, fluocinonide, halcinonide, triamcinolone acetonide, ciclesonide, deflazacort, formocortal, fludroxycortide, flunisolide and fluocinolone acetonide, those of the (beta)methasone-type (Group C), such as beclomethasone, betamethasone, betamethasone dipropionate and betamethasone valerate, dexamethasone, fluocortolone, halortietasorie, mometasone and mometasone furoate, alclometasone and alclometasone dipropionate, clobetasol and clobetasol propionate, clobetasone and clobetasone butyrate, clocortolone, desoximetasone, diflorasone, difluocortolone, fluclorolone, flumetasone, fluocortin, fluprednidene and fluprednidene acetate, fluticasone, fluticasone furoate and fluticasone propionate, meprednisone, paramethasone, prednylidene, rimexolone and ulobetasol; those of the progesterone-type, such as flugestone, fluorometholone, medrysone and prebediolone acetate, and progesterone derivatives (progestins), such as chlormadinone acetate, cyproterone acetate, medrogestone, medroxyprogesterone acetate, megestrol acetate and segesterone acetate; as well as other corticosteroids, such as cortivazol and 6-methyl-11β,17β-dihydroxy-17α-(1-propynyl)androsta-1,4,6-trien-3-one.

Preferred corticosteroids include cortisone, prednisone, prednisolone, methylprednisolone and, especially, dexamethasone.

Further, therapeutic agents that may be used in conjunction with compounds of the invention or pharmaceutically acceptable salts thereof include H2 receptor blockers, anticoagulants, anti-platelet drugs, as well as statins, antimicrobial agents and anti-allergic/anti-asthmatic drugs.

H2 receptor blockers that may be mentioned include famotidine. Anticoagulants that may be mentioned include heparin and low-molecular-weight heparins (e.g. bemiparin, nadroparin, reviparin, enoxaparin, parnaparin, certoparin, dalteparin, tinzaparin); directly acting oral anticoagulants (e.g. dabigatran, argatroban, i-ivai-oxaban, apixaban, edoxaban, betrixaban, darexaban, otamixaban, letaxaban, eribaxaban, hirudin, lepirudin and bivalirudin); coumarin type vitamin K antagonists (e.g. coumarin, acenocoumarol, phenprocoumon, atromentin and phenindione) and synthetic pentasaccharide inhibitors of factor Xa (e.g. fondaparinux, idraparinux and idrabiotaparinux). Anti-platelet drugs that may be mentioned include irreversible cyclooxygenase inhibitors (e.g. aspirin and triflusal); adenosine diphosphate receptor inhibitors (e.g. cangrelor, clopidogrel, prasugrel, ticagrelor and ticlopidine); phosphodiesterase inhibitors (e.g. cilostazol); protease-activated receptor-1 antagonists (e.g. vorapaxar); glycoprotein IIB/IIIA inhibitors (e.g. abciximab, eptifibatide and tirofiban); adenosine reuptake inhibitors (e.g. dipyridamole); and thromboxane inhibitors (e.g. terutroban, ramatroban, seratrodast and picotamide). Statins that may be mentioned include atorvastatin, simvastatin and rosuvastatin. Antimicrobial agents that may be mentioned include azithromycin, ceftriaxone, cefuroxime, doxycycline, fluconazole, piperacillin, tazobactam and teicoplanin. Anti-allergic/anti-asthmatic drugs that may be mentioned include chlorphenamine, levocetirizine and montelukast.

Subjects may thus also (and/or may be already) be receiving one or more of any of the other therapeutic agents mentioned above, by which we mean receiving a prescribed dose of one or more of those other therapeutic agents, prior to, in addition to, and/or following, treatment with compounds of the invention or pharmaceutical acceptable salts thereof.

When compounds of the invention are "combined" with other therapeutic agents in the aforementioned, the active ingredients may be administered together in the same formulation, or administered separately (simultaneously or sequentially) in different formulations.

Such combination products provide for the administration of compounds of the invention in conjunction with the other therapeutic agent, and may thus be presented either as separate formulations, wherein at least one of those formulations comprises a compound of the invention, and at least one comprises the other therapeutic agent, or may be presented (i.e. formulated) as a combined preparation (i.e. presented as a single formulation including a compound of the invention and the other therapeutic agent).

Thus, there is further provided:
(1) a pharmaceutical formulation including a compound of the invention; a therapeutic agent that is known to be metabolized by a CYP enzyme, such as any of those mentioned hereinbefore; and a pharmaceutically-acceptable excipient (e.g. adjuvant, diluent or carrier), which formulation is hereinafter referred to as a "combined preparation"; and
(2) a kit of parts comprising components:
   (A) a pharmaceutical formulation including a compound of the invention in admixture with a pharmaceutically-acceptable adjuvant, diluent or carrier; and
   (B) a pharmaceutical formulation including a therapeutic agent that is known to be metabolized by a CYP enzyme, such as any of those mentioned hereinbefore, in admixture with a pharmaceutically-acceptable adjuvant, diluent or carrier,
which components (A) and (B) are each provided in a form that is suitable for administration in conjunction with the other.

The therapeutic agents that are known to be metabolised by a CYP enzyme, present in the pharmaceutical formulations or kits of parts according to the invention, are as defined in the claims.

In a further non-claimed aspect of the disclosure, there is provided a process for the preparation of a combined preparation as hereinbefore defined, which process comprises bringing into association a compound of the invention, the other therapeutic agent, and at least one (e.g. pharmaceutically-acceptable) excipient.

In a further non-claimed aspect of the disclosure, there is provided a process for the preparation of a kit-of-parts as hereinbefore defined, which process comprises bringing into association components (A) and (B). As used herein, references to bringing into association will mean that the two components are rendered suitable for administration in conjunction with each other.

Thus, in relation to the process for the preparation of a kit-of-parts as hereinbefore defined, by bringing the two components "into association with" each other, we include that the two components of the kit-of-parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Thus, there is further provided (not part of the claimed invention) a kit of parts comprising:
(I) one of components (A) and (B) as defined herein; together with
(II) instructions to use that component in conjunction with the other of the two components.

The kits of parts described herein may comprise more than one formulation including an appropriate quantity/dose of a compound of the invention, and/or more than one formulation including an appropriate quantity/dose of the other therapeutic agent, in order to provide for repeat dosing. If more than one formulation (comprising either active compound) is present, such formulations may be the same, or may be different in terms of the dose of either compound, chemical composition(s) and/or physical form(s).

With respect to the kits of parts as described herein, by "administration in conjunction with", we include that respective formulations comprising a compound of the invention and other therapeutic agent are administered, sequentially, separately and/or simultaneously, over the course of treatment of the relevant condition.

Thus, in respect of the combination product according to the invention, the term "administration in conjunction with" includes that the two components of the combination product (compound of the invention and other therapeutic agent) are administered (optionally repeatedly), either together, or sufficiently closely in time, to enable a beneficial effect for the patient, that is greater, over the course of the treatment of the relevant condition, than if either a formulation comprising compound of the invention, or a formulation comprising the other agent, are administered (optionally repeatedly) alone, in the absence of the other component, over the same course of treatment. Determination of whether a combination provides a greater beneficial effect in respect of, and over the course of treatment of, a particular condition will depend upon the condition to be treated or prevented, but may be achieved routinely by the skilled person.

Further, in the context of a kit of parts according to the invention, the term "in conjunction with" includes that one or other of the two formulations may be administered (optionally repeatedly) prior to, after, and/or at the same time as, administration of the other component. When used in this context, the terms "administered simultaneously" and "administered at the same time as" include that individual doses of the relevant compound of the invention and other therapeutic agent are administered within 48 hours (e.g. 24 hours) of each other.

Pharmaceutical compositions/formulations, combination products and kits as described herein may be prepared in accordance with standard and/or accepted pharmaceutical practice.

Thus, in a further non-claimed aspect of the disclosure, there is provided a process for the preparation of a pharmaceutical composition/formulation, as hereinbefore defined, which process comprises bringing into association certain compounds of the invention, as hereinbefore defined, with one or more pharmaceutically-acceptable excipient (e.g. adjuvant, diluent and/or carrier).

In further non-claimed aspects of the disclosure, there is provided a process for the preparation of a combination product or kit-of-parts as hereinbefore defined, which process comprises bringing into association certain compounds of the invention, as hereinbefore defined, with the other therapeutic agent that is useful in the treatment of the relevant disease or disorder, and at least one pharmaceutically-acceptable excipient.

Certain compounds of the invention may be novel and/or not previously used in human medicine. Thus, in a further aspect of the invention, there is provided a compound of the invention (i.e. of formula I) as hereinbefore defined, or a pharmaceutically-acceptable salt thereof, provided that when Z represents -O-; R¹ represents methyl; R² and R³ both represent H; and R⁴ represents n-butyl, then R⁵ does not represent isobutyl.

Preferred compounds of the invention include those in which:
Z represents -O-;
R¹ represents a C₁₋₃ alkyl group (such as methyl, ethyl or propyl (e.g. *n*-propyl), preferably methyl or ethyl), optionally substituted by up to three halogen atoms (e.g. CH₂F or CH₂CF₃);
R² and R³ independently represent H or a C₁₋₃ alkyl group (such as methyl, ethyl or propyl (e.g. *n*-propyl)), optionally substituted by up to three halogen atoms (e.g. CH₂F or CH₂CF₃);
R⁴ represents a C₁₋₄ alkyl group (such as methyl, ethyl, propyl (e.g. *n*-propyl) or butyl (e.g. *n*-butyl)), optionally substituted by up to three halogen atoms; a C₁₋₆ alkoxy group, optionally substituted, or more preferably terminated, by up to three halogen atoms; aryl, optionally substituted by one or more halogen atoms (e.g. phenyl, fluorophenyl or trifluorophenyl); C₁₋₃ alkylaryl; or C₁₋₃ alkylheteroaryl;
R⁵ represents a C₁₋₄ alkyl group, optionally substituted or more preferably terminated by up to three fluorine atoms.

More preferred compounds of the invention include those in which:
Z represents -O-;
R¹ represents methyl or ethyl, optionally substituted by up to three fluorine atoms (e.g. CH₂F or CH₂CF₃);
R² and R³ independently represent methyl or ethyl or, more preferably, H;
R⁴ represents a C₁₋₄ alkyl group (such as methyl, ethyl, propyl (e.g. *n*-propyl) or butyl (e.g. *n*-butyl)), optionally substituted, or more preferably terminated, by up to three fluorine atoms; phenyl; C₁₂ alkylaryl; or C₁₋₂ alkylheteroaryl;
R⁵ represents butyl, more preferably isobutyl, optionally substituted or more preferably terminated by up to three fluorine atoms;

Particularly preferred compounds of the invention include those in which:
Z represents -O-,
R¹ represents ethyl or, more preferably, methyl;
R² represents methyl or, more preferably, H;
R³ represents H;
R⁴ represents an ethyl, propyl (e.g. *n*-propyl) or butyl (e.g. *n*-butyl) group, optionally substituted, or more preferably terminated, by up to three fluorine atoms; phenyl; benzyl; or pyridine-2-ylmethyl;
R⁵ represents butyl, more preferably isobutyl.

Thus, particular preferred compounds of the invention that may be mentioned include:
butyl (5-isobutyl-3-(4-((2-methyl-1H-imidazol-1-yl)methyl)phenyl)thiophen-2-yl)sulfonyl-carbamate;
butyl (3-(4-((2-ethyl-1H-imidazol-1-yl)methyl)phenyl)-5-isobutylthiophen-2-yl)sulfonyl-carbamate;
ethyl (5-isobutyl-3-(4-((2-methyl-1H-imidazol-1-yl)methyl)phenyl)thiophen-2-yl)sulfonyl-carbamate.

Particular novel compounds of the invention that may be mentioned include:
butyl (3-(4-((2-ethyl-1H-imidazol-1-yl)methyl)phenyl)-5-isobutylthiophen-2-yl)sulfonyl-carbamate;
ethyl (5-isobutyl-3-(4-((2-methyl-1H-imidazol-1-yl)methyl)phenyl)thiophen-2-yl)sulfonyl-carbamate.

Compounds are named according to IUPAC nomenclature generated by the program ChemDraw Ultra 12.0.

More preferred compounds of the invention include the compounds of the examples described hereinafter.

For purposes of interpreting this specification, the following definitions will apply and whenever appropriate, terms used in the singular will also include the plural and *vice versa.*

Pharmaceutically acceptable salts include acid addition salts and base addition salts. Such salts may be formed by conventional means, for example by reaction of a free acid or a free base form of a compound of the invention with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared using techniques known to those skilled in the art, such as by exchanging a counter-ion of a compound of the invention in the form of a salt with another counter-ion, for example using a suitable ion exchange resin.

Particular acid addition salts that may be mentioned include carboxylate salts such as formate, acetate, benzoate, oxalate, fumarate, maleate and the like, sulfonate salts such as methanesulfonate, ethanesulfonate, toluenesulfonate and the like, halide salts such as hydrochloride, hydrobromide and the like, sulfate and phosphate salts such as sulfate or phosphate and the like.

Particular base addition salts that may be mentioned include salts formed with alkali metals (such as Li, Na and K salts), alkaline earth metals (such as Mg and Ca salts), or other metals (such as Al and Zn salts) amine bases (such as ammonia, ethylenediamine, ethanolamine, diethanolamine, triethanolamine, tromethamine). More particularly, base addition salts that may be mentioned include Mg, Ca and, most particularly, K and Na salts.

Compounds of the invention may exist as solids, and thus the scope of the invention includes all amorphous, crystalline and part crystalline forms thereof, and may also exist as oils. Where compounds of formula I exist in crystalline and part crystalline forms, such forms may include solvates, which are included in the scope of the invention.

Compounds of the invention may also exist in solution (i.e. in solution in a suitable solvent). For example, compounds of formula I may exist in aqueous solution, in which case compounds of the invention may exist in the form of hydrates.

Compounds of the invention may contain double bonds and, unless otherwise indicated, may thus exist as E (*entgegen*) and Z (*zusammen*) geometric isomers about each individual double bond. Unless otherwise specified, all such isomers and mixtures thereof are included within the scope of the invention.

Compounds of the invention may also exhibit tautomerism. All tautomeric forms and mixtures thereof are included within the scope of the invention (particularly those of sufficient stability to allow for isolation thereof).

Compounds of the invention may also contain one or more asymmetric carbon atoms and may therefore exhibit optical and/or diastereoisomerism (i.e. existing in enantiomeric or diastereomeric forms). Diastereoisomers may be separated using conventional techniques, e.g. chromatography or fractional crystallisation. The various stereoisomers (i.e. enantiomers) may be isolated by separation of a racemic or other mixture of the compounds using conventional, e.g. fractional crystallisation or HPLC, techniques. Alternatively the desired enantiomer or diastereoisomer may be obtained from appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution; for example, with a homochiral acid followed by separation of the diastereomeric derivatives by conventional means such as chromatography), or by reaction with an appropriate chiral reagent or chiral catalyst, all of which methods and processes may be performed under conditions known to the skilled person. Unless otherwise specified, all stereoisomers and mixtures thereof are included within the scope of the invention.

As used herein, the term "halogen", when used herein, includes fluorine, chlorine, bromine and iodine.

Unless otherwise specified, C₁₋₆ alkyl groups (e.g. C₁₋₃ alkyl groups), and the alkyl parts of C₁₋₆ alkoxy, C₁₋₆ alkoxy-C₁₋₆ alkyl, C₁₋₆ alkylaryl, C₁₋₆ alkylheteroaryl groups (where, in all cases, 5 is the upper limit of the range), defined herein may be straight-chain or, when there is a sufficient number (i.e. a minimum of two or three, as appropriate) of carbon atoms, be branched-chain, and/or cyclic (so forming a C₃₋₆ cycloalkyl group). When there is a sufficient number (i.e. a minimum of four) of carbon atoms, such groups may also be part-cyclic (so forming a C₄₋₆ partial cycloalkyl group). For example, cycloalkyl groups that may be mentioned include cyclopropyl, cyclobutyl, cyclopentyl and cyclohexyl. Similarly, part-cyclic alkyl groups (which may also be referred to as "part-cycloalkyl" groups) that may be mentioned include cyclopropylmethyl. When there is a sufficient number of carbon atoms, such groups may also be multicyclic (e.g. bicyclic or tricyclic) and/or spirocyclic.

Particular alkyl groups that may be mentioned include straight chain (i.e. not branched and/or cyclic) alkyl groups. For example, C₁₋₆ alkyl groups, and the alkyl parts of C₁₋₆ alkoxy groups, include but are not limited to *n*-butyl, *sec*-butyl, isobutyl, *tert*-butyl; propyl, such as *n*-propyl, 2-methylpropyl or isopropyl; ethyl; and methyl.

For the avoidance of any doubt, the point of attachment of the C₁₋₅ alkyl groups, and the alkyl parts of C₁₋₆ alkoxy-C₁₋₆ alkyl, C₁₋₆ alkylaryl, C₁₋₆ alkylheteroaryl groups, is *via* the alkyl part of such groups.

For the avoidance of doubt, alkoxy groups are attached to the rest of the molecule *via* the oxygen atom in that group and alkoxyalkyl groups are attached to the rest of the molecule *via* the alkyl part of that group.

Unless otherwise specified, alkoxy refers to an O-alkyl group in which the term "alkyl" has the meaning(s) given above.

As used herein, references to heteroatoms will take their normal meaning as understood by one skilled in the art. Particular heteroatoms that may be mentioned include phosphorus, selenium, silicon, boron, oxygen, nitrogen and sulfur (e.g. oxygen, nitrogen and sulfur, such as oxygen and nitrogen).

As may be used herein, references to "heteroaryl" (which may also be referred to as heteroaromatic) rings or groups may refer to heteroaromatic groups containing one or more heteroatoms (such as one or more heteroatoms selected from oxygen, nitrogen and/or sulfur). Such heteroaryl groups may comprise one, two, or three rings, of which at least one is aromatic (which aromatic ring(s) may or may not contain the one or more heteroatom). Substituents on heteroaryl/heteroaromatic groups may, where appropriate, be located on any suitable atom in the ring system, including a heteroatom (e.g. on a suitable N atom).

The point of attachment of heteroaryl/heteroaromatic groups may be *via* any atom in the ring system including (where appropriate) a heteroatom. Bicyclic heteroaryl/heteroaromatic groups may comprise a benzene ring fused to one or more further aromatic or non-aromatic heterocyclic rings, in which instances, the point of attachment of the polycyclic heteroaryl/heteroaromatic group may be *via* any ring including the benzene ring or the heteroaryl/heteroaromatic or heterocyclyl ring.

For the avoidance of doubt, the skilled person will understand that heteroaryl groups that may form part of compounds of the invention are those that are chemically obtainable, as known to those skilled in the art. Various heteroaryl groups will be well-known to those skilled in the art, such as pyridinyl, pyrrolyl, furanyl, thiophenyl, oxadiazolyl, thiadiazolyl, thiazolyl, oxazolyl, pyrazolyl, triazolyl, tetrazolyl, isoxazolyl, isothiazolyl, imidazolyl, imidazopyrimidinyl, imidazothiazolyl, thienothiophenyl, pyrimidinyl, furopyridinyl, indolyl, azaindolyl, pyrazinyl, pyrazolopyrimidinyl, indazolyl, pyrimidinyl, quinolinyl, isoquinolinyl, quinazolinyl, benzofuranyl, benzothiophenyl, benzoimidazolyl, benzoxazolyl, benzothiazolyl, benzotriazolyl and purinyl.

As stated above, heteroaryl includes polycyclic (e.g. bicyclic) groups in which one ring is aromatic (and the other may or may not be aromatic). Hence, other heteroaryl groups that may be mentioned include groups such as benzo[1,3]dioxolyl, benzo[1,4]dioxinyl, dihydrobenzo[*d*]isothiazole, 3,4-dihydrobenz[1,4]oxazinyl, dihydrobenzothiophenyl, indolinyl, *5H*,*6H,7H*-pyrrolo[1,2-*b*]pyrimidinyl, 1,2,3,4-tetrahydroquinolinyl, thiochromanyl and the like.

As may be used herein, the term aryl may refer to C₆₋₁₄ (e.g. C₆₋₁₀) aromatic groups. Such groups may be monocyclic or bicyclic and, when bicyclic, be either wholly or partly aromatic. C₆₋₁₀ aryl groups that may be mentioned include phenyl, naphthyl, 1,2,3,4-tetrahydronaphthyl, indanyl, and the like (e.g. phenyl, naphthyl, and the like). Aromatic groups may be depicted as cyclic groups comprising therein a suitable number of double bonds to allow for aromaticity.

The skilled person will understand that aryl groups that may form part of compounds of the invention are those that are chemically obtainable, as known to those skilled in the art.

For the avoidance of doubt, the point of attachment of substituents on aryl groups may be *via* any suitable carbon atom of the ring system.

The present invention also embraces isotopically-labelled compounds of the present invention which are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Hence, the compounds of the invention also include deuterated compounds, i.e. compounds of the invention in which one or more hydrogen atoms are replaced by the hydrogen isotope deuterium.

In cases in which the identity of two or more substituents in a compound of the invention may be the same, the actual identities of the respective substituents are not in any way interdependent. For example, in the situation in which two or more halo groups are present, those groups may be the same or different (e.g. two chloro groups or a fluoro and a chloro group). Similarly, where two or more alkyl groups are present, the groups in question may be the same or different in terms of their number of carbon atoms and/or whether they are linear, branched, unsaturated or otherwise.

Further, when it is specified that a substituent is itself optionally substituted by one or more substituents (e.g. phenyl optionally substituted by one or more groups independently selected from halo), these substituents where possible may be positioned on the same or different atoms. Such optional substituents may be present in any suitable number thereof (e.g. the relevant group may be substituted with one or more such substituents, such as one such substituent).

Where groups are referred to herein as being optionally substituted it is specifically contemplated that such optional substituents may be not present (i.e. references to such optional substituents may be removed), in which case the optionally substituted group may be referred to as being unsubstituted.

Unless otherwise specified, substituents (whether optional or otherwise) may be located at any point on a group to which they may be attached. In this respect, alkyl and alkoxy groups (for example) that may be substituted by one or more substituents may also be terminated by such substituents (by which we mean located at the terminus of an e.g. alkyl or alkoxy chain).

For the avoidance of doubt, in cases in which the identity of two or more substituents in a compound of formula I may be the same, the actual identities of the respective substituents are not in any way interdependent. For example, in the situation in which R² and R³ are both C₁₋₃ alkyl, the C₁₋₃ alkyl groups in question may be the same or different.

The skilled person will appreciate that compounds of the invention that are the subject of this invention include those that are obtainable, i.e. those that may be prepared in a stable form. That is, compounds of the invention include those that are sufficiently robust to survive isolation, e.g. from a reaction mixture, to a useful degree of purity.

Compounds of formula I may be made in accordance with techniques well known to those skilled in the art, for example as described hereinafter.

According to a further aspect of the invention there is provided a process for the preparation of a compound of formula I, which process comprises:
(i) reaction of a compound of formula II, 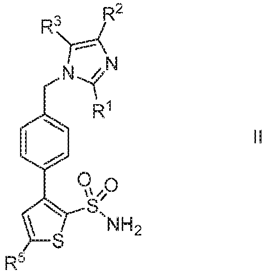
   wherein R¹, R², R³ and R⁵ are as hereinbefore defined, with a compound of formula III, 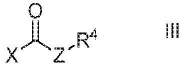
   wherein R⁴ and Z are as hereinbefore defined, and X represents a suitable leaving group, such as halo (e.g. chloro or bromo), for example at around room temperature or above (e.g. up to 60-70°C) in the presence of a suitable base (e.g. pyrollidinopyridine, pyridine, triethylamine, tributylamine, trimethylamine, N-ethyldiisopropylamine, dimethylaminopyridine, di-isopropylamine, 1,8-diazabicyclo[5.4.0]undec-7-ene or mixtures thereof) and an appropriate solvent (e.g. pyridine, dichloromethane, chloroform, tetrahydrofuran, dimethylformamide, or toluene).

Compounds of formula II may be prepared by reaction of a compound of formula IV, 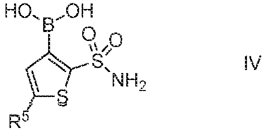
wherein R⁵ is as hereinbefore defined, or a *N*-potected derivative thereof, with a compound of formula V, 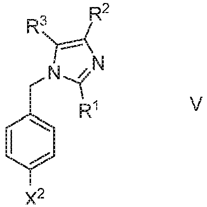
wherein X² represents a suitable leaving group, such as trimethylsulphonate, or halo, such as iodo or bromo, and R¹, R² and R³ are as hereinbefore defined, for example in the presence of an appropriate coupling catalyst system (e.g. a palladium catalyst, such as Pd(PPh₃)₄ or Pd(OAc)₂/ligand (wherein the ligand may be, for example, PPh₃, P(*o*-Tol)₃ or 1,1'-bis(diphenylphosphino)ferrocene)) and a suitable base (e.g. sodium hydroxide, sodium carbonate, potassium carbonate, caesium carbonate, triethylamine or di-iso-propylamine), as well as a suitable solvent system (e.g. toluene, ethanol, dimethoxymethane, dimethylformamide, ethylene glycol dimethyl ether, water, dioxane or mixtures thereof). This reaction (which is not part of the claimed invention) may be carried out at above room temperature (e.g. at the reflux temperature of the solvent system that is employed). If a protected version of a compound of formula IV is employed, this reaction may be followed by deprotection of the SO₂NH-group under standard conditions, for example as described hereinafter.

Compounds of formula II may alternatively be prepared by reaction of a compound of formula VI, 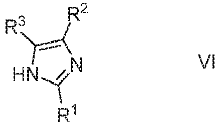
wherein R¹, R² and R³ are as hereinbefore defined with a compound of formula VII, 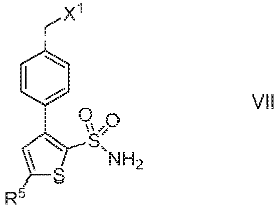
wherein R⁵ is as hereinbefore defined and X¹ represents a suitable leaving group such as halo (e.g. chloro or bromo, in particular, bromo), or an N-protected derivative thereof, for example at around or below room temperature in the presence of a suitable base (e.g. pyridine) and an appropriate organic solvent (e.g. toluene) (this reaction is not part of the claimed invention). If a protected version of a compound of formula VII is employed, this reaction may be followed by deprotection of the SO₂NH-group under standard conditions, for example as described hereinafter. Additionally, compounds of formula II may be prepared in this way for example according, or analogously, to processes described in *inter alia* UK patent application GB 2281298.

Compounds of formula V may be prepared by standard techniques, for example by way of reaction of a compound of formula VI as hereinbefore defined with a compound of formula VIII, 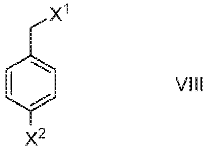 wherein X¹ and X² are as hereinbefore defined, for example under similar conditions to those described hereinbefore in respect of preparation of compounds of formula II (this reaction is not part of the claimed invention). Compounds of formula VII are known in the art. For example, they may be prepared according, or analogously, to processes described in *inter alia* US patent number 5,312,820, UK patent application GB 2281298, and/or international patent application WO 02/096883.

Compounds of formula IV are known in the art. For example, they may be prepared according, or analogously, to processes described in *inter alia* international patent application WO 02/096883.

Otherwise, compounds of formulae II to IV, VI, VII and VIII are either commercially available, are known in the literature, or may be obtained either by analogy with the processes described herein, or by conventional synthetic procedures, in accordance with standard techniques, from readily-available starting materials using appropriate reagents and reaction conditions.

It will be appreciated by those skilled in the art that, in the processes described above and hereinafter, the functional groups of intermediate compounds may need to be protected by protecting groups.

Functional groups that are desirable to protect include sulphonamido, amido, amino and aldehyde. Suitable protecting groups for sulphonamido, amido and amino include tert-butyloxycarbonyl, benzyloxycarbonyl, 2-trimethylsilylethoxycarbonyl (Teoc) or tert-butyl. Suitable protecting groups for aldehyde include alcohols, such as methanol or ethanol, and diols, such as 1,3-propanediol or, preferably, 1,2-ethanediol (so forming a cyclic acetal). The protection and deprotection of functional groups may take place before or after a reaction in the above-mentioned schemes.

Protecting groups may be applied and removed in accordance with techniques that are well-known to those skilled in the art and as described hereinafter. For example, protected compounds/intermediates described herein may be converted chemically to unprotected compounds using standard deprotection techniques. The type of chemistry involved will dictate the need, and type, of protecting groups as well as the sequence for accomplishing the synthesis. The use of protecting groups is fully described in *"*Protective Groups in Organic Synthesis", 3rd edition, T.W. Greene & P.G.M. Wutz, Wiley-Interscience (1999).

When used herein in relation to a specific value (such as an amount), the term "about" (or similar terms, such as "approximately") will be understood as indicating that such values may vary by up to 10% (particularly, up to 5%, such as up to 1%) of the value defined. It is contemplated that, at each instance, such terms may be replaced with the notation "±10%", or the like (or by indicating a variance of a specific amount calculated based on the relevant value). It is also contemplated that, at each instance, such terms may be deleted.

Compounds of the invention have the advantage that they are more potent than, and/or are stable to metabolic hydrolysis, and/or do not inhibit the CYP enzymes mentioned hereinbefore.

The compounds of the invention may also have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties than compounds known in the prior art, whether for use in the treatment of IPF or otherwise. Such effects may be evaluated clinically, objectively and/or subjectively by a health care professional, a treatment subject or an observer.

### Examples

The invention will be further described by reference to the following examples, which are not intended to limit the scope of the invention.

In the event that there is a discrepancy between nomenclature and any compounds depicted graphically, then it is the latter that presides (unless contradicted by any experimental details that may be given or unless it is clear from the context).

### Experimental procedures

Starting materials and intermediates used in the synthesis of compounds described herein are commercially available or can be prepared by the methods described herein or by methods known in the art.

Experiments were generally carried out under inert atmosphere (nitrogen or argon), particularly in cases where oxygen- or moisture-sensitive reagents or intermediates were used.

Mass spectrometry data are reported from liquid chromatography-mass spectrometry (LC-MS). Chemical shifts for NMR data are expressed in parts per million (ppm, δ) referenced to residual peaks from the deuterated solvent used.

For syntheses referencing general procedures, reaction conditions (such as length of reaction or temperature) may vary. In general, reactions were followed by thin layer chromatography or LC-MS, and subjected to work-up when appropriate. Purifications may vary between experiments: in general, solvents and the solvent ratios used for eluents/gradients were chosen to provide an appropriate R_{f} and/or retention time. Some products were purified using supercritical fluid chromatography, for example on a reversed phase column using solvent combinations with mobile phase A: CO₂ and B: MeOH/H₂O/NH₃.

### Example 1

### Butyl (5-isobutyl-3-(4-((2-methyl-1H-imidazol-1-yl)methyl)phenyl)thiophen-2-yl)sulfonyl-carbamate

### (a) N-tert-butyl-5-isobutyl-3-[4-[(2-methylimidazol-1-yl)methyl]phenyl]thiophene-2-sulfon-amide

1-[(4-Bromophenyl)methyl]-2-methyl-imidazole (1.25 g; 5 mmol; prepared using procedures similar to literature for 1-[(4-bromophenyl)methyl]-2-methyl-imidazole (see e.g. international patent application WO 2002/096883)), 5-isobutyl-2-(*tert-*butylaminosulfonyl)-3-thiopheneboronic acid (1.59 g, 5 mmol; prepared as described in international patent application WO 2002/096883), K₂CO₃ (2.06 g; 15 mmol) and Pd(PPh₃)₄ (144 mg; 120 µmol) were added to dioxane (100 mL) and water (10 mL). The mixture was heated to 95°C overnight under a nitrogen atmosphere. Most of the solvent was evaporated. Water was added (50 mL) and the product was extracted with diethyl ether (2 x 50 mL). After drying and evaporation, the isolated sub-title compound was used directly in the next step.

### (b) 5-Isobutyl-3-[4-[(2-methylimidazol-1-yl)methyl]phenyl]thiophene-2-sulfonamide

The sub-title compound from step (a) above (1.36 g) was dissolved in dichloromethane (30 mL). Boron trichloride (15 mL, 1M in dichloromethane) was added and the solution was stirred 2 hours at room temperature. Na₂CO₃ (sat., 20 mL) was added and the product was extracted with ethyl acetate (40 mL). After drying and evaporation the isolated sub-title compound was used directly in the next step.

### (c) Butyl N-[[5-isobutyl-3-[4-[(2-methylimidazol-1-yl)methyl]phenyl]-2-thienyl]sulfonyl]-carbamate

The sub-title compound from step (b) above (1.2 g) and N-ethyldiisopropylamine (2.57 g; 20 mmol) were dissolved in dichloromethane. Butyl chloroformate (2.04 g; 15 mmol) was added slowly at room temperature. After 1 hour, water was added and the product was extracted with diethyl ether. The sub-title compound was isolated using column chromatography from dichloromethane-methanol (90:10). The amount of sub-title compound isolated was 1.05 g (yield 43% over the three reaction steps).

¹H-NMR (CDCl₃): 0.85 (t, 3H), 0.99 (d, 6H), 1.24 (m, 2H), 1.48 (m, 2H), 1.94 (m, 1H), 2.54 (s, 3H), 2.69 (d, 2H), 3.99 (t, 2H), 5.11 (s, 2H), 6.72 (s, 1H), 6.81 (m, 2H), 7.07 (d, 2H), 7.60 (d, 2H)

MS (M+H): experimental 490.1824 calculated 490.1834.

### Example 2

### Ethyl (5-isobutyl-3-(4-((2-methyl-1H-imidazol-1-yl)methyl)phenyl)thiophen-2-yl)sulfonyl-carbamate

The title compound is prepared by a process that is analogous to the one described in Example 1, with the exception that ethyl chloroformate is employed in the final step.

### Example 3

### Butyl (3-(4-((2-ethyl-1H-imidazol-1-yl)methyl)phenyl)-5-isobutylthiophen-2-yl)sulfonyl-carbamate

The title compound is prepared by a process that is analogous to the one described in Example 1, with the exception that 1-[(4-bromophenyl)methyl]-2-ethylimidazole is employed in the first step.

### Biological Assays

The biological activity of example compounds as described herein above was assessed (and compared to C21) using the following biological assays.

### Binding to AT1 and AT2 receptor

Compounds were evaluated for binding to the human recombinant AT2 and AT1 receptor according to Eurofins protocol ITEM26 and ITEM24 using a radiometric scintillation assay.

Briefly, recombinant protein was incubated for 2-4 h at 37°C with test compounds at concentration 1,10,100 and 1000 nM for the AT2 receptor and 1 and 10 µM for the AT1 receptor. ¹²⁵I(sar1,IIe8)-AT-II was used as a ligand for the AT1 receptor and ¹²⁵ICGP 42112A was used as a ligand for the AT2 receptor. Percent inhibition of control specific binding was calculated according to 100 - (measured specific binding/control specific binding) x 100.

| | AT2 IC₅₀ [nM] | AT1 IC₅₀ [nM] |
|---|---|---|
| Example 1 | 0.53 | >1000 |
| C21 | 5.1 | > 10000 |

### CYP inhibition

Compounds were evaluated at 10 µM for inhibition of the main cytochrome P450 isoforms (CYP1A, CYP2B6, CYP2C8, CYP2C9, CYP2C19, CYP2D6 and CYP3A4) using isoform-specific substrates incubated with human liver microsomes (Eurofins protocol ITEMG232). The following substrates were used; CYP1A phenacetin, CYP2B6 bupropion, CYP2C8 paclitaxel and amodiaquine, CYP2C9 diclofenac, CYP2C19 omeprazole, CYP2D6 dextromethorphan, CYP3A midazolam and testosterone.

At the end of the incubation, the formation of metabolite was monitored by HPLC-MS/MS as the peak area response.

| | CYP1A Inh % | CYP2B6 Inh % | CYP2C19 Inh % | CYP2C8 Inh % | CYP2C9 Inh % | CYP2D6 Inh % |
|---|---|---|---|---|---|---|
| Example 1 | 2.5 | 17.6 | 43.0 | 37.5 | 58.9 | 22.4 |
| C21 | 90.9 | 48.8 | 96.0 | 80.4 | 99.0 | 81.2 |

| | CYP3A4 midazolam Inh % | CYP3A4&5 testosterone Inh % |
|---|---|---|
| Example 1 | ∼0 | 13.5 |
| C21 | 95.2 | 94.2 |

### Abbreviations

The following abbreviations may be used herein.

| | |
|---|---|
| DMSO | dimethylsulfoxide |
| NMR | nuclear magnetic resonance |
| rt | room temperature |
| THF | tetrahydrofuran |

## Claims

1. A compound of formula I, 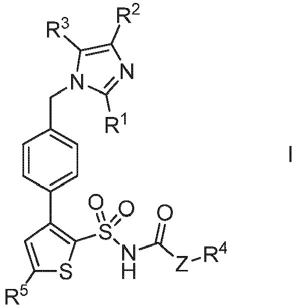 wherein:
Z represents -O- or a direct bond;
R¹ represents C₁₋₃ alkyl, optionally substituted by one or more halogen atoms;
R² and R³ each independently represent H or C₁₋₃ alkyl, optionally substituted by one or more halogen atoms;
R⁴ represents C₁₋₆ alkyl or C₁₋₆ alkoxy, each of which is optionally substituted with one or more halogen atoms, or
R⁴ represents aryl, C₁₋₆ alkylaryl, heteroaryl or C₁₋₆ alkylheteroaryl, each of which is optionally substituted by one or more substituents selected from halogen, -CH₂F, -OCF₃, C₁₋₆ alkyl, and C₁₋₆ alkoxy;
R⁵ represents C₁₋₆ alkyl, C₁₋₆ alkoxy or C₁₋₆ alkoxy-C₁₋₆alkyl, each of which is optionally substituted by one or more halogen atoms,
or a pharmaceutically-acceptable salt thereof, for use in the treatment of a disease or condition in which activation of AT2 receptors is desired or required but in which inhibition of CYPs is not desired, wherein said disease or condition is selected from the group consisting of an interstitial lung disease, an autoimmune disease, a chronic kidney disease, pulmonary hypertension, an obstructive airway disease, a viral respiratory tract infection and pneumonia as a consequence thereof and infarction.

2. A compound for use as defined in Claim 1, wherein:
a. Z represents -O-;
b. R¹ represents methyl or ethyl;
c. R² and R³ independently represent H, methyl or ethyl;
d. R⁴ represents a C₁₋₄ alkyl group, optionally substituted or terminated by up to three fluorine atoms; phenyl; C₁₋₃ alkylaryl; or C₁₋₃ alkylheteroaryl; and/or
e. R⁵ represents a C₁₋₄ alkyl group, optionally substituted or more preferably terminated by up to three fluorine atoms.

3. A compound for use as defined in any one of the preceding claims, which is:
butyl (5-isobutyl-3-(4-((2-methyl-1H-imidazol-1-yl)methyl)phenyl)thiophen-2-yl)sulfonyl-carbamate;
butyl (3-(4-((2-ethyl-1H-imidazol-1-yl)methyl)phenyl)-5-isobutylthiophen-2-yl)sulfonylcarbamate; or
ethyl (5-isobutyl-3-(4-((2-methyl-1H-imidazol-1-yl)methyl)phenyl)thiophen-2-yl)sulfonyl-carbamate.

4. A compound as defined in Claim 1 or Claim 2, or a pharmaceutically-acceptable salt thereof, provided that when: Z represents -O-; R¹ represents methyl; R² and R³ both represent H; and R⁴ represents n-butyl, then R⁵ does not represent isobutyl.

5. A compound as claimed in Claim 4, which is:
butyl (3-(4-((2-ethyl-1H-imidazol-1-yl)methyl)phenyl)-5-isobutylthiophen-2-yl)sulfonylcarbamate; or
ethyl (5-isobutyl-3-(4-((2-methyl-1H-imidazol-1-yl)methyl)phenyl)thiophen-2-yl)sulfonyl-carbamate.

6. A compound as defined in Claim 4 or Claim 5, for use as a pharmaceutical.

7. A pharmaceutical formulation comprising a compound as defined in Claim 4 or Claim 5 in admixture with a pharmaceutically-acceptable, adjuvant, diluent or carrier.

8. A compound as defined in Claim 4 or Claim 5, for use in the treatment of a disease or condition in which activation of AT2 receptors is desired or required but in which inhibition of CYPs is not desired as defined in Claim 1.

9. A pharmaceutical formulation comprising a compound as defined in any one of Claims 1 to 5; a therapeutic agent that is known to be metabolized by a CYP enzyme; and a pharmaceutically-acceptable adjuvant, diluent or carrier, wherein the therapeutic agent is selected from the group consisting of pirfinidone, naproxen, propranolol, riluzole, tizanidine, warfarin, celecoxib, clopidogrel, irbesartan, meloxicam, piroxicam, torsemide, cyclophosphamide, indomethacin, atorvastatin, cilostazol, cyclosporine, deflazacort, hydrocortisone, lidocaine, selexipag, sildenafil, simvastatin, and pharmaceutically-acceptable salts of any of those agents.

10. A kit of parts comprising:
(A) a pharmaceutical formulation including a compound as defined in any one of Claims 1 to 5 in admixture with a pharmaceutically-acceptable adjuvant, diluent or carrier; and
(B) a pharmaceutical formulation including a therapeutic agent that is known to be metabolized by a CYP enzyme as defined in Claim 9, in admixture with a pharmaceutically-acceptable adjuvant, diluent or carrier,
which components (A) and (B) are each provided in a form that is suitable for administration in conjunction with the other.

11. A formulation as claimed in any one Claims 7 or 9, or a kit of parts as claimed in Claim 10, for use in the treatment of a disease or condition in which activation of AT2 receptors is desired or required but in which inhibition of CYPs is not desired as defined in Claim 1.

12. A compound for use as claimed in any one of Claims 1 to 3 or 8, a formulation for use or a kit of parts for use as claimed in Claim 11, wherein the disease or condition to be treated is selected from the group:
rheumatoid arthritis, diabetic nephropathy, pulmonary arterial hypertension, chronic obstructive airway disease, virally-induced pneumonia and myocardial infarction.

13. A compound for use, a formulation for use, or a kit of parts for use, as claimed in Claim 12, wherein the disease is:
(i) idiopathic pulmonary fibrosis; and/or
(ii) sarcoidosis.

14. A compound for use, a formulation for use, or a kit of parts for use, as claimed in Claim 13, wherein the therapeutic agent that is known to be metabolized by a CYP enzyme is pirfenidone, or a pharmaceutically-acceptable salt thereof.

15. A process for the preparation of a compound of formula I as defined in Claim 4 or Claim 5, which process comprises:
(i) reaction of a compound of formula II, 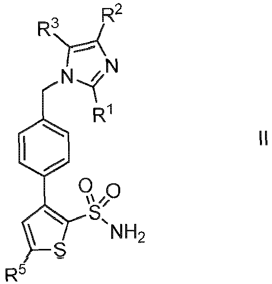
wherein R¹, R², R³ and R⁵ are as defined in Claim 4, with a compound of formula III, 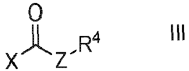
Wherein R4 and Z are as defined in claim 4; and X represents a suitable leaving group.

## Patentansprüche

1. Verbindung der Formel I, 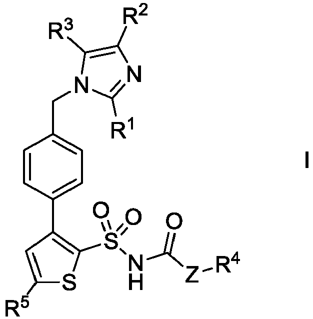 wobei:
Z für -0- oder eine direkte Bindung steht;
R¹ für C₁₋₃-Alkyl steht, optional substituiert durch ein oder mehrere Halogenatome;
R² und R³ jeweils für H oder C₁₋₃-Alkyl, optional substituiert durch ein oder mehrere Halogenatome, stehen;
R⁴ für C₁₋₆-Alkyl oder C₁₋₆-Alkoxy steht, die jeweils optional mit einem oder mehreren Halogenatomen substituiert sind, oder
R⁴ für Aryl, C₁₋₆-Alkylaryl, Heteroaryl oder C₁₋₆-Alkylheteroaryl steht, die jeweils optional durch einen oder mehrere Substituenten substituiert sind, ausgewählt aus Halogen, -CH₂F, -OCF₃, C₁₋₆-Alkyl und C₁₋₆-Alkoxy;
R⁵ für C₁₋₆-Alkyl, C₁₋₆-Alkoxy oder C₁₋₆-Alkoxy-C₁₋₆-Alkyl steht, die jeweils optional mit einem oder mehreren Halogenatomen substituiert sind,
oder ein pharmazeutisch verträgliches Salz davon zur Verwendung bei der Behandlung einer Krankheit oder eines Zustands, bei der/dem eine Aktivierung von AT2-Rezeptoren erwünscht oder erforderlich ist, bei der/dem jedoch eine Hemmung von CYPs nicht erwünscht ist, wobei die Krankheit oder der Zustand ausgewählt ist aus der Gruppe, bestehend aus einer interstitiellen Lungenerkrankung, einer Autoimmunerkrankung, einer chronischen Nierenerkrankung, pulmonaler Hypertonie, einer obstruktiven Atemwegserkrankung, einer viralen Atemwegsinfektion und Lungenentzündung als Folge davon und Infarkt.

2. Verbindung zur Verwendung nach Anspruch 1, wobei:
a. Z für -0- steht;
b. R¹ für Methyl oder Ethyl steht;
c. R² und R³ unabhängig voneinander für H, Methyl oder Ethyl stehen;
d. R⁴ für eine C₁₋₄-Alkylgruppe steht, die optional mit bis zu drei Fluoratomen; Phenyl; C₁₋₃ Alkylaryl; oder C₁₋₃ Alkylheteroaryl substituiert oder terminiert ist; und/oder
e. R⁵ für eine optional substituierte oder noch bevorzugter durch bis zu drei Fluoratome terminierte C₁₋₄-Alkylgruppe steht.

3. Verbindung zur Verwendung nach einem der vorstehenden Ansprüche, die Folgendes ist:
Butyl-(5-isobutyl-3-(4-((2-methyl-1H-imidazol-1-yl)methyl)phenyl)thiophen-2-yl)sulfonyl-carbamat;
Butyl-(3-(4-((2-Ethyl-1H-imidazol-1-yl)methyl)phenyl)-5-isobutylthiophen-2-yl)sulfonyl-carbamat; oder
Ethyl-(5-isobutyl-3-(4-((2-methyl-1H-imidazol-1-yl)methyl)phenyl)thiophen-2-yl)sulfonyl-carbamat.

4. Verbindung nach Anspruch 1 oder 2 oder ein pharmazeutisch verträgliches Salz davon, vorausgesetzt, dass wenn: Z für -0- steht; R¹ für Methyl steht; R² und R³ beide für H stehen; und R⁴ für n-Butyl steht, dann steht R⁵ nicht für Isobutyl.

5. Verbindung nach Anspruch 4, die Folgendes ist:
Butyl-(3-(4-((2-Ethyl-1H-imidazol-1-yl)methyl)phenyl)-5-isobutylthiophen-2-yl)sulfonyl-carbamat; oder
Ethyl-(5-isobutyl-3-(4-((2-methyl-1H-imidazol-1-yl)methyl)phenyl)thiophen-2-yl)sulfonyl-carbamat.

6. Verbindung nach Anspruch 4 oder 5, zur Verwendung als Arzneimittel.

7. Pharmazeutische Formulierung, umfassend eine Verbindung nach Anspruch 4 oder 5 in Mischung mit einem pharmazeutisch verträglichen Adjuvans, Verdünnungsmittel oder Träger.

8. Verbindung nach Anspruch 4 oder 5 zur Verwendung bei der Behandlung einer Krankheit oder eines Zustands, bei der/dem die Aktivierung von AT2-Rezeptoren erwünscht oder erforderlich ist, bei der/dem aber die Hemmung von CYPs nach Anspruch 1 nicht erwünscht ist.

9. Pharmazeutische Formulierung, umfassend eine Verbindung nach einem der Ansprüche 1 bis 5; ein therapeutisches Mittel, von dem bekannt ist, dass es durch ein CYP-Enzym metabolisiert wird; und ein pharmazeutisch verträgliches Adjuvans, Verdünnungsmittel oder Träger, wobei das therapeutische Mittel ausgewählt ist aus der Gruppe, bestehend aus Pirfinidon, Naproxen, Propranolol, Riluzol, Tizanidin, Warfarin, Celecoxib, Clopidogrel, Irbesartan, Meloxicam, Piroxicam, Torsemid, Cyclophosphamid, Indomethacin, Atorvastatin, Cilostazol, Cyclosporin, Deflazacort, Hydrocortison, Lidocain, Selexipag, Sildenafil, Simvastatin und pharmazeutisch verträglichen Salzen eines dieser Mittel.

10. Teilesatz, umfassend:
(A) eine pharmazeutische Formulierung, die eine Verbindung nach einem der Ansprüche 1 bis 5 in Mischung mit einem pharmazeutisch verträglichen Adjuvans, Verdünnungsmittel oder Träger einschließt; und
(B) eine pharmazeutische Formulierung, die ein therapeutisches Mittel einschließt, von dem bekannt ist, dass es durch ein CYP-Enzym nach Anspruch 9 definiert, metabolisiert wird, in Mischung mit einem pharmazeutisch verträglichen Adjuvans, Verdünnungsmittel oder Träger, wobei Komponente (A) und (B) jeweils in einer Form bereitgestellt sind, die für eine Verabreichung in Verbindung mit der anderen geeignet ist.

11. Formulierung nach einem der Ansprüche 7 oder 9 oder ein Teilesatz nach Anspruch 10 zur Verwendung bei der Behandlung einer Krankheit oder eines Zustands, bei der/dem eine Aktivierung von AT2-Rezeptoren erwünscht oder erforderlich ist, bei der/dem aber eine Hemmung von CYPs gemäß der Definition in Anspruch 1 nicht erwünscht ist.

12. Verbindung zur Verwendung nach einem der Ansprüche 1 bis 3 oder 8, eine Formulierung zur Verwendung oder ein Teilesatz zur Verwendung nach Anspruch 11, wobei die zu behandelnde Krankheit oder der zu behandelnde Zustand ausgewählt ist aus der Gruppe:
rheumatoide Arthritis, diabetische Nephropathie, pulmonale arterielle Hypertonie, chronisch obstruktive Atemwegserkrankung, viral bedingte Lungenentzündung und Myokardinfarkt.

13. Verbindung zur Verwendung, eine Formulierung zur Verwendung oder ein Teilesatz zur Verwendung nach Anspruch 12, wobei die Krankheit Folgendes ist:
(i) idiopathische Lungenfibrose; und/oder
(ii) Sarkoidose.

14. Verbindung zur Verwendung, eine Formulierung zur Verwendung oder ein Teilesatz zur Verwendung nach Anspruch 13, wobei das therapeutische Mittel, von dem bekannt ist, dass es durch ein CYP-Enzym metabolisiert wird, Pirfenidon oder ein pharmazeutisch verträgliches Salz davon ist.

15. Prozess zur Herstellung einer Verbindung der Formel I nach Anspruch 4 oder 5, wobei der Prozess Folgendes umfasst:
(i) Reaktion einer Verbindung der Formel II, 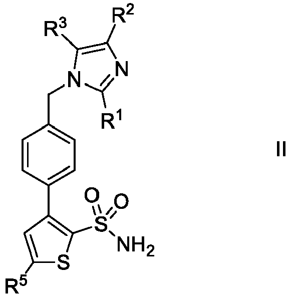
wobei R¹, R², R³ und R⁵ nach Anspruch 4 definiert sind, mit einer Verbindung der Formel III, 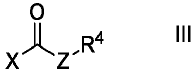
wobei R4 und Z nach Anspruch 4 definiert sind; und X eine geeignete Abgangsgruppe darstellt.

## Revendications

1. Composé de formule I, 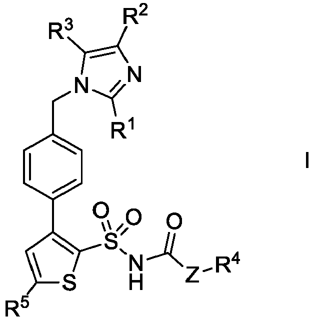 dans lequel :
Z représente -0- ou une liaison directe ;
R¹ représente un alkyle en C₁₋₃, facultativement substitué avec un ou plusieurs atomes d'halogène ;
R² et R³ représentent chacun indépendamment H ou un alkyle en C₁₋₃, facultativement substitué avec un ou plusieurs atomes d'halogène ;
R⁴ représente un alkyle en C₁₋₆ ou un alcoxy en C₁₋₆, chacun étant facultativement substitué avec un ou plusieurs atomes d'halogène, ou
R⁴ représente un aryle, un (alkyle en C₁₋₆) aryle, un hétéroaryle ou un (alkyle en C₁₋₆)hétéroaryle, chacun étant facultativement substitué avec un ou plusieurs substituants sélectionnés parmi un halogène, -CH₂F, -OCF₃, un alkyle en C₁₋₆ et un alcoxy en C₁₋₆ ;
R⁵ représente un alkyle en C₁₋₆, un alcoxy en C₁₋₆ ou un alcoxy en C₁₋₆-alkyle en C₁₋₆, chacun étant facultativement substitué avec un ou plusieurs atomes d'halogène,
ou un sel pharmaceutiquement acceptable de celui-ci, pour une utilisation dans le traitement d'une maladie ou d'une affection dans laquelle l'activation de récepteurs AT2 est souhaitée ou requise mais dans laquelle l'inhibition de CYP n'est pas souhaitée, dans lequel ladite maladie ou affection est sélectionnée dans le groupe consistant en une maladie pulmonaire interstitielle, une maladie auto-immune, une maladie rénale chronique, une hypertension pulmonaire, une maladie obstructive des voies respiratoires, une infection virale des voies respiratoires et une pneumonie en conséquence de celle-ci et un infarctus.

2. Composé pour une utilisation selon la revendication 1, dans lequel :
a. Z représente -0- ;
b. R¹ représente un méthyle ou un éthyle ;
c. R² et R³ représentent indépendamment H, un méthyle ou un éthyle ;
d. R⁴ représente un groupe alkyle en C₁₋₄, facultativement substitué avec ou terminé par jusqu'à trois atomes de fluor ; un phényle ; un (alkyle en C₁₋₃)aryle ; ou un (alkyle en C₁₋₃) hétéroaryle ; et/ou
e. R⁵ représente un groupe alkyle en C₁₋₄, facultativement substitué avec ou de manière davantage préférée terminé par jusqu'à trois atomes de fluor.

3. Composé pour une utilisation selon l'une quelconque des revendications précédentes, qui est :
le (5-isobutyl-3-(4-((2-méthyl-1H-imidazol-1-yl)-méthyl)phényl)thiophèn-2-yl)sulfonyl-carbamate de butyle ;
le (3-(4-((2-éthyl-1H-imidazol-1-yl)méthyl)phényl)-5-isobutylthiophèn-2-yl)sulfonyl-carbamate de butyle ; ou
le (5-isobutyl-3-(4-((2-méthyl-1H-imidazol-1-yl)-méthyl)phényl)thiophèn-2-yl)sulfonyl-carbamate d'éthyle.

4. Composé selon la revendication 1 ou la revendication 2, ou un sel pharmaceutiquement acceptable de celui-ci, à condition que lorsque : Z représente -0- ; R¹ représente un méthyle ; R² et R³ représentent tous les deux H ; et R⁴ représente un n-butyle, alors R⁵ ne représente pas un isobutyle.

5. Composé selon la revendication 4, qui est :
le (3-(4-((2-éthyl-1H-imidazol-1-yl)méthyl)phényl)-5-isobutylthiophèn-2-yl)sulfonyl-carbamate de butyle ; ou
le (5-isobutyl-3-(4-((2-méthyl-1H-imidazol-1-yl)-méthyl)phényl)thiophèn-2-yl)sulfonyl-carbamate d'éthyle.

6. Composé selon la revendication 4 ou la revendication 5, pour une utilisation comme produit pharmaceutique.

7. Formulation pharmaceutique comprenant un composé selon la revendication 4 ou la revendication 5 en mélange avec un adjuvant, diluant ou support pharmaceutiquement acceptable.

8. Composé selon la revendication 4 ou la revendication 5, pour une utilisation dans le traitement d'une maladie ou d'une affection dans laquelle l'activation de récepteurs AT2 est souhaitée ou requise mais dans laquelle l'inhibition de CYP n'est pas souhaitée comme défini dans la revendication 1.

9. Formulation pharmaceutique comprenant un composé selon l'une quelconque des revendications 1 à 5 ; un agent thérapeutique qui est connu pour être métabolisé par une enzyme CYP ; et un adjuvant, diluant ou support pharmaceutiquement acceptable, dans laquelle l'agent thérapeutique est sélectionné dans le groupe consistant en par la pirfinidone, le naproxène, le propranolol, le riluzole, la tizanidine, la warfarine, le célécoxib, le clopidogrel, l'irbésartan, le méloxicam, le piroxicam, le torsémide, le cyclophosphamide, l'indométhacine, l'atorvastatine, le cilostazol, la cyclosporine, le déflazacort, l'hydrocortisone, la lidocaïne, le sélexipag, le sildénafil, la simvastatine, et les sels pharmaceutiquement acceptables de l'un quelconque de ces agents.

10. Kit de parties comprenant :
(A) une formulation pharmaceutique comprenant un composé selon l'une quelconque des revendications 1 à 5 en mélange avec un adjuvant, diluant ou support pharmaceutiquement acceptable ; et
(B) une formulation pharmaceutique comprenant un agent thérapeutique qui est connu pour être métabolisé par une enzyme CYP telle que définie dans la revendication 9, en mélange avec un adjuvant, diluant ou support pharmaceutiquement acceptable,
lesquels composants (A) et (B) sont chacun fournis sous une forme qui est appropriée pour une administration en conjonction avec l'autre.

11. Formulation selon l'une quelconque des revendications 7 ou 9, ou kit de parties selon la revendication 10, pour une utilisation dans le traitement d'une maladie ou d'une affection dans laquelle l'activation de récepteurs AT2 est souhaitée ou requise mais dans laquelle l'inhibition de CYP comme défini dans la revendication 1.

12. Composé pour une utilisation selon l'une quelconque des revendications 1 à 3 ou 8, formulation pour une utilisation ou kit de parties pour une utilisation selon la revendication 11, dans lequel la maladie ou l'affection à traiter est sélectionnée dans le groupe suivant :
polyarthrite rhumatoïde, néphropathie diabétique, hypertension artérielle pulmonaire, maladie obstructive chronique des voies respiratoires, pneumonie d'origine virale et infarctus du myocarde.

13. Composé pour une utilisation, formulation pour une utilisation ou kit de parties pour une utilisation, selon la revendication 12, dans lequel la maladie est :
(i) une fibrose pulmonaire idiopathique ; et/ou
(ii) une sarcoïdose.

14. Composé pour une utilisation, formulation pour une utilisation ou kit de parties pour une utilisation, selon la revendication 13, dans lequel l'agent thérapeutique qui est connu pour être métabolisé par une enzyme CYP est la pirfénidone, ou un sel pharmaceutiquement acceptable de celle-ci.

15. Procédé de préparation d'un composé de formule I selon la revendication 4 ou la revendication 5, lequel procédé comprend :
(i) la réaction d'un composé de formule II, 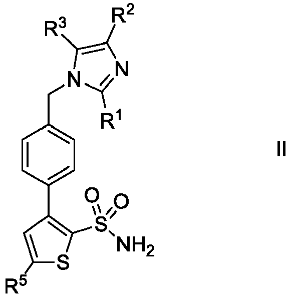
dans lequel R¹, R², R³ et R⁵ sont tels que définis dans la revendication 4, avec un composé de formule III, 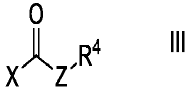
dans lequel R4 et Z sont tels que définis dans la revendication 4 ; et X représente un groupe partant approprié.
